(19) Europäisches Patentamt
European Patent Office
Office européen des brevets

(11) EP 1 242 173 B1

(12) **FASCICULE DE BREVET EUROPEEN**

(45) Date de publication et mention de la délivrance du brevet:
**06.08.2003 Bulletin 2003/32**

(21) Numéro de dépôt: **00990861.7**

(22) Date de dépôt: **29.12.2000**

(51) Int Cl.7: **B01J 13/00**, B01J 35/00

(86) Numéro de dépôt international:
**PCT/FR00/03755**

(87) Numéro de publication internationale:
**WO 01/049405 (12.07.2001 Gazette 2001/28)**

(54) **DISPERSION COLLOIDALE AQUEUSE A BASE D'AU MOINS UN COMPOSE D'UN METAL ET D'UN COMPLEXANT, PROCEDE DE PREPARATION ET UTILISATION**

WÄSSRIGE, BASISCHE, KOLLOIDALE DISPERSION, DIE MINDESTENS EIN METALL UND EIN KOMPLEX ENTHÄLT; IHR HERSTELLUNGSVERFAHREN SOWIE VERWENDUNG

AQUEOUS COLLOIDAL DISPERSION BASED ON AT LEAST A METAL COMPOUND AND A COMPLEXING AGENT, PREPARATION METHOD AND USE

(84) Etats contractants désignés:
**AT BE CH CY DE DK ES FI FR GB GR IE IT LI LU MC NL PT SE TR**

(30) Priorité: **30.12.1999 FR 9916786**

(43) Date de publication de la demande:
**25.09.2002 Bulletin 2002/39**

(73) Titulaire: **RHODIA CHIMIE**
**92512 Boulogne Billancourt Cedex (FR)**

(72) Inventeur: **CHANE-CHING, Jean-Yves**
**F-95600 Eaubonne (FR)**

(74) Mandataire: **Bernasconi, Jean et al**
**c/o Cabinet Lavoix,**
**2, Place d'Estienne d'Orves**
**75441 Paris Cédex (FR)**

(56) Documents cités:
**EP-A- 0 448 441**
**EP-A- 0 504 030**
**US-A- 5 128 114**
**US-A- 5 643 497**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 242 173 B1

Printed by Jouve, 75001 PARIS (FR)

## Description

La présente invention a trait à des dispersions colloïdales aqueuses à base d'au moins un composé d'un métal et d'un complexant. Elle concerne également le procédé de préparation et les utilisations de ces dispersions.

Les dispersions colloïdales (ou sols) d'oxydes et/ou d'oxydes hydratés (hydroxydes) métalliques, tout particulièrement les sols de zircone ou d'oxydes de titane, sont bien connus de l'homme du métier et leurs modes d'obtention ont été largement décrits dans l'art antérieur. En ce qui concerne les sols d'oxyde de zircone, par exemple on pourra notamment se référer au Journal of Gel Science Technology , vol. 1, p. 223 (1994). On peut également citer l'article de Chemical Materials , vol. 10, pp. 3217-3223 (1998), en ce qui concerne les sols d'oxyde de titane.

Par ailleurs, il est à noter que, selon la nature de l'élément métallique mis en oeuvre, ces suspensions peuvent présenter un grand intérêt pour des applications dans le domaine de la catalyse, ou encore dans le domaine de la protection anti-UV. Cependant, il est à souligner que, dans ce type d'applications, notamment de façon à augmenter la surface spécifique des colloïdes, ou de façon à obtenir des suspensions transparentes, on a besoin de sols dits "de type nanométrique" comprenant des colloïdes extrêmement fins, et possédant avantageusement des dimensions de l'ordre de quelques nanomètres à quelques dizaines de nanomètres.

Or, il est souvent difficile d'obtenir de tels sols. On observe en effet souvent des cinétiques rapides de formation des particules, ce qui entraîne des difficultés pour arrêter la polycondensation minérale au stade de particules nanométriques. On obtient donc généralement *in fine* des particules de taille trop importante présentant une forte tendance à la décantation.

Des dispersions aqueuses colloïdales, dont les particules présentent un diamètre moyen supérieur à 10 nm, et leurs procédés du préparation sont connus de EP-A-0 504 030 et EP-A-0 448 441.

Le but de la présente invention est de résoudre de telles difficultés et d'obtenir des sols stables comprenant des colloïdes de dimensions nanométriques.

Plus précisément, la présente invention a pour objet une dispersion colloïdale aqueuse au sein de laquelle le diamètre moyen des particules colloïdales est au plus de 6 nm, ladite dispersion comprenant :

(1) au moins un composé d'un métal M, ledit métal M étant un métal majoritaire au sein de la dispersion, choisi dans le groupe constitué de l'aluminium, du zirconium et des métaux de la classification périodique dont le numéro atomique est compris entre 22 et 31; et

(2) au moins un complexant présentant un $pK_s$(M) (cologarithme de la constante de dissociation du complexe formé par le complexant et le cation dudit métal M) supérieur à 2.

De façon particulièrement préférée, ledit métal M est choisi parmi l'aluminium, le titane, le fer et le zirconium.

L'invention concerne également un procédé de préparation des dispersions précédentes, qui est caractérisé en ce qu'il comporte les étapes consistant à :

(a) former un mélange aqueux comprenant au moins un sel dudit métal M et au moins un complexant, avec un taux molaire de complexant par rapport au(x) sel(s) introduit(s) compris entre 0,1 et 3

(b) ajouter une base au mélange ainsi formé ; et

(c) chauffer le mélange ainsi obtenu.

D'autres caractéristiques, détails et avantages de l'invention apparaîtront encore plus complètement à la lecture de la description qui va suivre, ainsi que des divers exemples concrets mais non limitatifs destinés à l'illustrer.

La classification périodique des éléments à laquelle il est fait référence dans la présente description est celle publiée dans le Supplément au Bulletin de la Société Chimique de France n° 1 (janvier 1966).

Par "lanthanide", on entend les éléments du groupe constitué par l'yttrium et les éléments de la classification périodique de numéro atomique compris de façon inclusive entre 57 et 71.

Au sens de la présente invention, une dispersion colloïdale (ou sol) d'un composé d'un métal M, ou d'un métal M et d'un ou de plusieurs élément(s) des types précités, désigne tout système constitué de fines particules solides de dimensions colloïdales, à base généralement d'oxyde et/ou d'oxyde hydraté (hydroxyde) du métal M, ou, le cas échéant, d'oxyde et/ou d'oxyde hydraté (hydroxyde) du métal et du ou des autre(s) élément(s) et, éventuellement, de complexant, en suspension dans une phase liquide aqueuse, lesdites espèces pouvant en outre, éventuellement, contenir des quantités résiduelles d'ions liés ou adsorbés tels que par exemple des ions chlorures, des ions nitrates, des ions acétates, des ions citrates, des ions ammoniums, ou du complexant sous forme ionisée. On notera également que dans de telles dispersions, le métal M et le ou les autre(s) élément(s) peuvent se trouver soit totalement sous la

forme de colloïdes, soit simultanément sous la forme d'ions, d'ions complexés et sous la forme de colloïdes.

Les dispersions de l'invention sont spécifiquement des dispersions au sein desquelles le métal M est un métal présent en quantité majoritaire. Ainsi, il peut s'agir, selon un premier mode de réalisation, de dispersions uniquement à base d'un ou plusieurs composé(s) du métal M, mais les dispersions de l'invention peuvent aussi, selon un mode particulier, comprendre, en plus d'un ou plusieurs composé(s) du métal M, un ou plusieurs composé(s) d'un ou de plusieurs élément(s) métallique(s) additionnel(s) autre(s) que le métal M, choisi(s) parmi les lanthanides, l'aluminium et les éléments des groupes IVa, Va, VIa, VIIa, VIII, Ib et IIb de la classification périodique. Toutefois, selon ce mode particulier, le métal M reste toujours spécifiquement majoritaire au sein de la dispersion, c'est à dire que le rapport molaire (métal M) / (métal M+ autre(s) élément(s) métalliques(s)) est toujours au minimum égal à 50%, étant entendu que ce rapport est strictement supérieur à 50% dans le cas spécifique de la présence d'un composé d'un lanthanide.

En fait, et en particulier dans le cas de la présence additionnelle d'au moins un composé d'un lanthanide, le rapport molaire (métal M) / (métal M+autre(s) élément(s)) reste généralement strictement supérieur à 50%. Selon ce mode particulier, ce ratio molaire peut alors avantageusement être supérieur à 60%, et notamment supérieur à 70%. Ce ratio peut, en particulier être supérieur à 80%, voire à 90%.

Parmi les lanthanides particulièrement adaptés à ce mode particulier de réalisation de l'invention, on peut notamment citer l'yttrium, le cérium, le lanthane, le néodyme, le gadolinium et le samarium

Comme élément du groupe IVa, on peut citer notamment le titane et le zirconium.

A titre d'élément du groupe Va, on peut citer le vanadium.

Le chrome et le molybdène peuvent être plus particulièrement choisis comme éléments du groupe VIa, et le manganèse pour le groupe VIIa.

Comme élément du groupe VIII, on utilisera avantageusement le fer, le cobalt et le nickel. Dans ce même groupe, on peut mentionner également les métaux précieux comme le platine, l'iridium, le ruthénium, le rhodium et le palladium.

Le cuivre, l'argent et l'or d'une part et le zinc d'autre part peuvent être choisis en ce qui concerne les groupes Ib et IIb, respectivement.

Ainsi, on peut mentionner comme dispersions particulièrement avantageuses selon ce mode de réalisation, celles à base de l'association d'aluminium et de zinc, d'aluminium et de titane, de titane et de palladium, de fer et de titane ou encore de zirconium et de cérium.

Selon un aspect plus particulier, l'invention concerne également des dispersions dites "stoechiométriques". Ces dispersions spécifiques sont à base d'un métal M et d'un unique élément métallique additionnel A choisi parmi un des éléments métalliques précités, à l'exception d'un lanthanide, à savoir l'aluminium et les éléments des groupes IVa, Va, VIa, VIIa, VIII, Ib et IIb de la classification périodique, et sont spécifiquement caractérisées par un rapport molaire M/(M+A) qui, dans ce cas précis, est égal à 50%.

Comme dispersion selon ce mode de réalisation particulier, on peut citer à titre d'exemple les dispersions dites "$FeTiO_3$", à base d'oxyde de fer FeO et d'oxyde de titane $TiO_2$, en rapport équimolaire.

Quelle que soit la nature chimique exacte des composés métallique présents au sein des dispersions de l'invention, il est à souligner que ces dispersions sont spécifiquement des dispersions dans lesquelles, pour plus de 50% des colloïdes, le diamètre moyen est d'au plus 6 nm. De façon particulière, ce diamètre moyen peut être inférieur à 5 nm, et il peut notamment être compris entre 2 nm et 5 nm.

Les diamètres précités peuvent être déterminés notamment par comptage photométrique à partir d'une analyse par METHR (Microscopie Electronique par Transmission à Haute Résolution), complétée si nécessaire par cryomicroscopie.

Outre leur faible taille, les colloïdes des dispersions de l'invention présentent également un taux d'agrégation peu élevé. Les analyses par cryo-microscopie électronique à transmission montrent un taux d'agrégats de colloïdes faible, généralement inférieur à 10% en nombre, voire inférieur à 5% en nombre, c'est à dire que, sur l'ensemble des objets que l'on observe, généralement au plus 10% sont constitués de plusieurs particules agrégées.

Le terme "complexant" désigne au sens de l'invention un composé ou une molécule susceptible d'établir une liaison covalente ou iono-covalente avec le cation métallique et/ou le(s) cation(s) de l'autre (ou des autres) élément (s). Les complexants de la présente invention sont des complexants pour lesquels la ou les constante(s) de dissociation du ou des complexe(s) formé(s) avec le ou les ion(s) métallique(s) présent(s) est ou sont faible(s) (complexes stables). Les complexes considérés ici sont le ou les complexe(s) formé(s) par le ou les complexant(s) et le cation métallique et, le cas échéant, le ou les complexe(s) formé(s) par le complexant et le ou les élément(s) supplémentaire(s) précité(s).

A titre d'exemple pour l'équilibre donné ci-dessous (complexation d'un cation du métal M) :

$$\text{Complexe} \underset{}{\overset{K_s}{\rightleftarrows}} \text{Cation du métal M + Complexant} \quad ,$$

la constante $K_s(M)$ de dissociation du complexe est donnée par la formule :

$$K_s(M) = [\text{Cation du métal M}] \times [\text{Complexant}] / [\text{Complexe}]$$

Le $pK_s(M)$ est le cologarithme de $K_s(M)$, défini par $pK_s(M) = -\log K_s(M)$. Cette valeur dépend à la fois de la nature du cation métallique et de la nature du complexant et reflète la stabilité du complexe formé par ces deux espèces. En d'autres termes, plus le complexe est stable, plus la valeur de $pK_s(M)$ est élevée.

Les complexants mis en oeuvre dans le cadre de l'invention présentent vis à vis du ou des différents cation (s) métallique(s) avec le(s)quel(s) ils forment un ou des complexe(s) une ou des valeur(s) de $pK_s$ supérieure(s) à 2, et de préférence d'au moins 3.

La nature du complexant mis en oeuvre est donc à adapter en fonction de la nature du métal M et du ou des autre(s) élément(s) éventuellement présent(s).

Toutefois, il est à noter que, dans le cas général, le complexant peut être avantageusement choisi parmi les acides-alcools, ou les polyacides-alcools ou les sels de ces composés. Comme exemple d'acide-alcool particulièrement adapté, on peut citer l'acide glycolique ou l'acide lactique. Comme polyacide-alcool, on peut mentionner notamment l'acide malique et l'acide citrique.

Le complexant peut aussi être avantageusement choisi parmi les acides aliphatiques aminés, de préférence les polyacides aliphatiques aminés, ou leurs sels. Comme exemple de tels complexants, on peut citer l'acide éthylène-diamino-tétracétique ou l'acide nitrilo-tri-acétique ou encore le sel de sodium de l'acide glutamique N, N diacétique de formule $(NaOOC)CH_2CH_2\text{-}CH(COONa)\ N(CH_2COONa)_2$.

Comme autres complexants adaptés, on peut également citer les acides polyacryliques ou leurs sels, tels que le polyacrylate de sodium, et plus particulièrement ceux dont la masse moléculaire est comprise entre 2000 et 5000.

On notera enfin que, selon l'invention, un ou plusieurs complexants peuvent être présents dans la même dispersion.

Le taux de complexant présent au sein des suspensions de l'invention est déterminé par dosage chimique du carbone et du métal M présents dans la dispersion colloïdale. Ce taux est exprimé en nombre de moles de complexant par rapport au nombre total de moles de métal M et varie avantageusement entre 0,1 et 2 , et de façon particulièrement préférée entre 0,1 et 1,5.

Le taux de complexant présent dans les colloïdes est quant à lui déterminé par dosage chimique du carbone et du métal M présents dans le culot de centrifugation obtenu par ultracentrifugation de la dispersion à 50 000 tours par minute pendant 6 heures. Ce taux de complexant au sein des colloïdes, également exprimé en nombre de moles de complexant par rapport au nombre de moles de métal M, est avantageusement compris entre 0,1 et 2 , et plus préférentiellement entre 0,2 et 1.

Le calcul de ces taux s'applique à la somme des complexants si plusieurs complexants sont présents dans la dispersion, et à la somme des différents éléments métalliques présents si un ou plusieurs éléments supplémentaires sont présents en plus du métal M.

Les dispersions de l'invention peuvent présenter des valeurs de pH dans une gamme étendue, en particulier entre 3 et 10, et avantageusement entre 7 et 9, ce qui permet de les utiliser notamment dans les applications où un pH voisin de la neutralité est requis.

Les concentrations des dispersions de l'invention sont d'au moins 0,1 mole/l. Elles peuvent avantageusement être d'au moins 0,5 mole/l, voire supérieures à 1 mole/l. Ces concentrations sont exprimées en concentration du métal M ou, le cas échéant, en concentration du métal M et du ou des élément(s) précité(s). Cette concentration est déterminée après séchage et calcination sous air d'un volume donné de dispersion.

Le mode de préparation des dispersions de l'invention va maintenant être décrit.

La première étape (a) du procédé de préparation consiste à former un mélange aqueux comprenant au moins un sel du métal M et le complexant avec un taux molaire de complexant par rapport au(x) sel(s) introduit(s) compris entre 0,1 et 3. Ce mélange peut éventuellement comprendre en outre au moins un sel d'un ou plusieurs élément(s) des groupes cités plus haut dans le cas de la préparation des dispersions à base d'au moins un de ces éléments.

Selon un autre mode de mise en oeuvre, il est également possible dans ce cas de préparer d'une part un premier mélange avec au moins un sel du métal M et un premier complexant, et d'autre part un ou plusieurs autre(s) mélange(s) comprenant chacun un ou plusieurs sel(s) du ou des élément(s) métaltique(s) additionnel(s) potentiel(s) précité(s) et le même complexant, ou un complexant différent de celui utilisé dans le premier mélange, avec un taux molaire de complexant par rapport au(x) sel(s) introduit(s) compris entre 0,1 et 3.

Les sels peuvent être des sels d'acides inorganiques ou organiques, par exemple du type sulfate, nitrate, chlorure ou encore acétate. On notera que les chlorures, les nitrates et les acétates conviennent particulièrement bien. Comme sels de titane, fer, aluminium, ou zirconium, on peut utiliser plus particulièrement $TiOCl_2$, $Fe(NO_3)_3$, $Al(NO_3)_3$ et $ZrO(NO_3)_2$.

Dans le ou les mélange(s) de départ, le taux molaire de complexant par rapport au(x) sel(s) introduit(s) est compris entre 0,1 et 3 , et est avantageusement compris entre 0,25 et 2.

La deuxième étape (b) du procédé consiste à augmenter le pH du ou des mélange(s) préparé(s) lors de l'étape (a). On y ajoute donc une base. Comme base, particulièrement adaptée, on peut citer notamment les hydroxydes alcalins ou alcalino-terreux et l'ammoniaque. On peut également utiliser les amines secondaires ou tertiaires. Toutefois, les amines et l'ammoniaque peuvent être préférés dans la mesure où ces composés évitent les risques de pollution par des cations alcalins ou alcalino terreux.

L'ajout de la base se fait jusqu'à l'obtention d'un pH dont la valeur varie en fonction de la nature du métal M et, le cas échéant, du ou des élément(s) mis en oeuvre et de la nature et du taux de complexant. En particulier, il est à noter que le pH est d'autant plus faible que le taux de complexant est élevé. On ajoute généralement la base jusqu'à l'obtention d'une valeur de pH pour laquelle on commence à observer la dissolution du précipité qui se forme dans la première partie de l'étape d'addition. Dans le cas de la mise en oeuvre particulière décrite plus haut et dans laquelle on a préparé deux ou plusieurs mélanges de départ, on ajoute une base dans chacun des différents mélanges de façon séparée, on réunit ensuite ces mélanges et on ajuste éventuellement le pH si nécessaire.

La troisième étape (c) du procédé est un traitement thermique, appelé également thermohydrolyse, qui consiste à chauffer le mélange obtenu à l'issue de l'étape (b). La température de chauffe est généralement d'au moins 60°C, et de préférence d'au moins 100°C et elle peut s'élever jusqu'à la température critique du milieu réactionnel.

Ce traitement thermique peut être conduit, selon les conditions de températures retenues, soit sous pression normale atmosphérique, soit sous pression telle que par exemple la pression de vapeur saturante correspondant à la température du traitement thermique. Lorsque la température de traitement est choisie supérieure à la température de reflux du mélangé réactionnel (c'est à dire généralement supérieure à 100°C), on conduit alors généralement l'opération en introduisant le mélange aqueux dans une enceinte close (réacteur fermé plus couramment appelé autoclave), la pression nécessaire ne résultant alors que du seul chauffage du milieu réactionnel (pression autogène). Dans les conditions de températures données ci-dessus, et en milieux aqueux, on peut ainsi préciser, à titre illustratif, que la pression dans le réacteur fermé varie alors entre une valeur supérieure à 1 bar ($10^5$ Pa) et 165 bar (165. $10^5$ Pa), de préférence entre 1 bar ($10^5$ Pa) et 20 bar (20. $10^5$ Pa). Il est bien entendu également possible d'exercer une pression extérieure qui s'ajoute alors à celle consécutive au chauffage.

Le chauffage peut être conduit soit sous atmosphère d'air, soit sous atmosphère de gaz inerte, et de préférence dans ce cas sous atmosphère d'azote.

La durée du traitement n'est pas critique, et peut de ce fait varier dans de larges limites, avantageusement entre 1 et 48 heures, et de préférence entre 2 et 24 heures.

A l'issue du traitement, on obtient une dispersion selon l'invention.

Selon une variante de l'invention, la dispersion obtenue à l'issue de l'étape (c) peut être traitée par ultrafiltration, notamment de façon purifier et à concentrer la dispersion obtenue à l'issue de l'étape (c). Ce traitement peut avoir lieu immédiatement après ladite étape (c), ou ultérieurement.

Le cas échéant, l'ultrafiltration peut être effectuée notamment sous air ou sous atmosphère d'air et d'azote ou encore sous azote. Elle se déroule de préférence avec une eau dont le pH est ajusté au pH de la dispersion.

Les suspensions de l'invention peuvent également être soumises à d'autres étapes de concentration, et notamment à une concentration par évaporation. La température d'évaporation est alors définie de manière à éviter la floculation de la dispersion colloïdale. A titre indicatif, cette température est généralement comprise entre 20°C et 90°C et avantageusement entre 20°C et 60°C.

Les dispersions de l'invention peuvent être utilisées dans de nombreuses applications. On peut citer par exemple la catalyse notamment pour les réactions de trans-estérification ou de polycondensation, par exemple pour la production de polyéthylène téréphtalate, ou encore pour la réaction d'amidification, en particulier pour la synthèse de polyamide. Dans ce cas les dispersions sont utilisées dans la préparation de catalyseurs. Les dispersions peuvent aussi être mises en oeuvre pour leurs propriétés anti-UV par exemple dans la préparation de films de polymères protecteurs (du type acrylique ou polycarbonate par exemple) ou de compositions cosmétiques notamment dans la préparation de crèmes anti-UV. Enfin, elles peuvent également être utilisées pour le renfort de matrices polymères ou minérales.

Des exemples vont maintenant être donnés.

## EXEMPLE 1

Cet exemple concerne la préparation d'une dispersion colloïdale à base d'un composé d'aluminium selon l'invention.

(a) On a introduit dans un bécher 112,5 g (soit 0,3 mole) d'$Al(NO_3)_3$, 9 $H_2O$ (M=375 g/mol), et 31,5 g (soit 0,15 mole) d'acide citrique monohydraté (M=210,14 g/mol). On a ensuite additionné 120 g d'eau déminéralisée, puis

on a soumis le mélange obtenu à une agitation à une température de 25°C, de façon à réaliser une solution d'un volume total de 200 ml, avec un rapport molaire (complexant / aluminium) de 0,5.

(b) On a ensuite ajouté à cette solution, maintenue sous agitation à 25°C, une solution d'ammoniaque 10 M préalablement dosée et maintenue en atmosphère fermée, de façon à minimiser son évaporation. Cette addition a été réalisée à l'aide d'une pompe doseuse et avec un débit contrôlé de 0,5 ml par minute. Lors de cette addition d'ammoniaque, on a observé la formation d'une dispersion opaque blanche. Lorsqu'on a commencé à noter un éclaircissement de cette suspension, soit au bout de 3 heures et 45 minutes, on a arrêté l'addition. Le volume total d'ammoniaque ajouté était alors de 111,5 ml, et la valeur du pH de la dispersion obtenue de 8,3.

(c) La dispersion ainsi réalisée a alors été immédiatement transférée dans une bombe de Parr. Cette bombe de Parr a été placée dans une étuve préalablement portée à 120°C, et un traitement thermique de la dispersion à 120°C a été conduit durant 14 heures dans cette bombe de Parr jouant le rôle d'autoclave.

Après refroidissement, 120 ml de la dispersion d'oxy-hydroxyde d'aluminium ainsi obtenue ont été soumis à une ultrafiltration. Pour ce faire, on a additionné 240 ml d'eau déminéralisée aux 120 ml de la dispersion obtenue après traitement thermique. On a ultrafiltré la suspension obtenue sur une membrane de 3 KD jusqu'à obtenir un volume de dispersion de 120 ml. On a répété cette opération deux fois, à cela près que l'ultrafiltration de la dernière opération a été conduite jusqu'à un volume final de dispersion non pas de 120 ml, mais de 60 ml.

La teneur équivalente en $Al_2O_3$ déterminée par étuvage et calcination à une température de 900°C d'une aliquote de la dispersion est de 5,24 % en masse. La concentration en aluminium dans la dispersion obtenue après ultrafiltration est de 1,03 mol/l.

Par cryo-microscopie à transmission sur l'échantillon sans dilution, on observe des particules de dimension nanométrique parfaitement individualisées dont la taille moyenne est de 3 nm.

Par ailleurs, la dispersion obtenue est stable vis à vis de la décantation sur une période supérieure à 6 mois.

**EXEMPLE 2**

Cet exemple concerne une dispersion colloïdale de pH neutre, à base d'un composé de titane.

(a) On a introduit dans un bécher 16,7 g d'acide citrique (M=192 g/mol). On a ensuite additionné 404 g d'eau déminéralisée, puis on a additionné, sous agitation, et de façon instantanée, 78 g d'une solution de $TiOCl_2$, 2 HCl possédant une teneur en titane de 4,95 mol/l, c'est-à-dire de 3,19 mol/kg, de façon à réaliser une solution limpide à l'oeil et caractérisée par un rapport molaire (complexant / titane) de 0,35.

(b) On a ensuite ajouté à cette solution, maintenue sous agitation à une température de 25°C, 100 ml d'une solution d'ammoniaque 10M, à l'aide d'une pompe doseuse possédant un débit constant de 0,6 ml/ mn. Lors de cette addition d'ammoniaque, on a observé la formation d'un précipité, puis d'une dispersion opaque blanche. En fin d'addition, soit au bout de 2 heures et 45 minutes, on a obtenu une dispersion caractérisée par une teneur en titane de 0,45 mol/litre.

(c) La dispersion ainsi réalisée a alors été transférée dans une bombe de Parr. Cette bombe de Parr a été placée dans une étuve préalablement portée à 90°C. Un traitement thermique de la dispersion à 90°C a alors été conduit durant 14 heures dans la bombe de Parr jouant le rôle d'autoclave.

Après refroidissement, 120 ml de la dispersion de TiO2 ainsi obtenue ont été soumis à une ultrafiltration. Pour ce faire, on a additionné 240 ml d'eau déminéralisée aux 120 ml de la dispersion obtenue après traitement thermique. On a ultrafiltré la suspension obtenue sur une membrane de 3 KD jusqu'à obtenir un volume de dispersion de 120 ml. On a répété cette opération deux fois, à cela près que l'ultrafiltration de la dernière opération a été conduite jusqu'à un volume final de dispersion non pas de 120 ml, mais de 60 ml. On a alors obtenu une dispersion de pH égal à 6,5.

Le dosage par calcination d'une aliquote montre pour cette dispersion une concentration massique de 6% en $TiO_2$ après ultrafiltration.

Une concentration finale de la solution colloïdale a ensuite réalisée par évaporation. On a introduit dans un cristallisoir de diamètre 7 cm et de hauteur 4 cm, 55 ml de la dispersion colloïdale de $TiO_2$ obtenue à l'issu de l'étape d'ultrafiltration et on l'a soumise à une évaporation, à une température de 50°C pendant 6 heures. La concentration en $TiO_2$ de la dispersion issue de cette étape d'évaporation est de 12,3% en masse, soit de 1,53 mol/litre en titane.

Par cryo-microscopie électronique à transmission sur l'échantillon dilué jusqu'à une concentration massique 2% en $TiO_2$ , on observe des particules de dimensions nanométriques de $TiO_2$ parfaitement dont la taille moyenne est

de 3 nm.

La dispersion est stable vis à vis de la décantation sur une période supérieure à 6 mois.

**EXEMPLE 3**

Cet exemple concerne une dispersion colloïdale à base d'un composé de titane et d'un composé de cérium.

(a) On a introduit dans un bécher 13,68 g de $CeCl_3$ solide (de teneur équivalente en CeO2 de 45,8%, déterminée par calcination à 1000°C pendant une heure), puis 250 ml d'eau déminéralisée. Après mise sous agitation et dissolution du sel, on a additionné 46 ml d'une solution de $TiOCl_2$, 2HCl à 3,5 mol/l. Le volume de la solution a été complété à 500 ml avec de l'eau déminéralisée. A une aliquote de 400 ml, on a ajouté 11,6 g d'acide citrique (M= 192 g/mol), de façon à obtenir une solution caractérisée par un rapport molaire (Ti/Ce) de 4,4:1, soit par un rapport molaire Ti/(Ti+Ce) de 81%, et un rapport molaire complexant/(Ti+Ce) de 0,38.

(b) On a ensuite ajouté à cette solution, maintenue sous agitation à une température de 25°C, 97,4 g d'une solution d'ammoniaque 9,96 M, à l'aide d'une pompe doseuse, avec un débit contrôlé de 3 ml/mn.

(c) La suspension ainsi réalisée a été transférée dans une bombe de Parr. Cette bombe de Parr a été placée dans une étuve préalablement portée à 120°C. Un traitement thermique de la dispersion a alors été conduit pendant 16 heures à 120°C dans la bombe de Parr jouant le rôle d'autoclave.

On a laissé refroidir la dispersion obtenue à température ambiante. La valeur du pH de la dispersion obtenue est de 8,8.

Par cryo-microscopie en transmission, on observe des particules de dimension nanométrique parfaitement individualisées de taille moyenne de 3 nm.

Une aliquote de 27 g de la dispersion obtenue a été soumise à une ultracentrifugation à 50 000 tours/mn pendant 6 heures. On a séparé le culot (2,0 g) et la phase surnageante (24,94 g). Par dosage chimique du titane et du cérium, le rapport molaire (Ti/Ce) est de 5,6:1 dans le culot, on mesure un pourcentage de (Ti+Ce) sous forme colloïdale présent dans la dispersion déterminé de 92%. Par dosage du carbone, on détermine un pourcentage molaire citrate/ (Ti+Ce) de 0,20 dans les colloïdes.

**EXEMPLE 4**

Cet exemple concerne une dispersion colloïdale à base d'un composé du fer.

(a) On a introduit dans un bécher 20,2 g de $Fe(NO_3)_3$, x $H_2O$ (M=404 g/mol), et 19,2 g d'acide citrique(M=192 g/mol). On a ensuite ajusté le volume à 100ml avec de l'eau déminéralisée, puis on a soumis le mélange obtenu à une agitation à 25°C, de façon à réaliser une solution d'un volume total de 200 ml, avec un rapport molaire (complexant / Fe) égal à 2.

(b) On a ensuite ajouté à cette solution, maintenue sous agitation, une solution d'ammoniaque 10 M, à l'aide d'une pompe doseuse et à raison de 3 ml/mn, jusqu'à obtenir un pH de 3,7.

(c) La dispersion ainsi réalisée a alors été transférée dans une bombe de Parr où elle a été maintenue à 120° pendant 16 heures. Ce traitement thermique dans la bombe de Parr jouant le rôle d'autoclave, conduit à l'obtention d'une dispersion stable vis à vis de la décantation pendant une période supérieure à 6 mois.

**Revendications**

1. Dispersion colloïdale aqueuse, au sein de laquelle le diamètre moyen des particules colloïdales est au plus de 6 nm, ladite dispersion comprenant :

    (1) au moins un composé d'un métal M, ledit métal M étant un métal majoritaire au sein de la dispersion, choisi dans le groupe constitué de l'aluminium, du zirconium et des métaux de la classification périodique dont le numéro atomique est compris entre 22 et 31 ;

    (2) au moins un complexant présentant un $pK_s$(M) (cologarithme de la constante de dissociation du complexe

formé par le complexant et le cation dudit métal M) supérieur à 2.

2. Dispersion selon la revendication 1, **caractérisée en ce que** les particules colloïdales présentent un diamètre moyen compris entre 2 et 5 nm.

3. Dispersion selon la revendication 1 ou la revendication 2, **caractérisée en ce que** le métal M est choisi parmi l'aluminium, le titane, le fer et le zirconium.

4. Dispersion selon l'une des revendications précédentes, **caractérisée en ce que** le complexant est choisi parmi les acides-alcools ou polyacides-alcools, les acides aliphatiques aminés, les polyacides aliphatiques aminés, les acides polyacryliques ou les sels de ces composés.

5. Dispersion selon l'une des revendications précédentes, **caractérisée en ce que** le complexant présente un $pK_s$ d'au moins 3.

6. Dispersion selon l'une des revendications précédentes, **caractérisée en ce qu'**elle présente un pH compris entre 3 et 10.

7. Dispersion selon l'une des revendications 1 à 6, **caractérisée en ce qu'**elle présente une concentration en métal M d'au moins 0,5 mole/l.

8. Dispersion selon l'une des revendications précédentes, **caractérisée en ce que** le taux de complexant au sein de la suspension exprimé en nombre de moles de complexant par rapport au nombre de moles de métal M est compris entre 0,1 et 2.

9. Dispersion selon l'une des revendications précédentes, **caractérisée en ce que** le taux de complexant présent dans les colloïdes exprimé en nombre de moles de complexant par rapport au nombre de moles de métal M est compris entre 0,1 et 2.

10. Dispersion selon l'une des revendications 1 à 9, **caractérisée en ce qu'**elle comprend un ou plusieurs composé (s) d'un ou de plusieurs élément(s) métallique(s) additionnel(s) autre(s) que ledit métal M, choisi(s) parmi les lanthanides, l'aluminium et les éléments des groupes IVa, Va, VIa, VIIa, VIII, Ib et IIb de la classification périodique, le rapport molaire (métal M) / (métal M+autre(s) élément(s)) étant strictement supérieur à 50%.

11. Dispersion selon l'une des revendications 1 à 9, **caractérisée en ce qu'**elle comprend un composé d'un élément métallique additionnel A choisi parmi l'aluminium et les éléments des groupes IVa, Va, VIa, VIIa, VIII, Ib et IIb de la classification périodique, le rapport molaire M/(M+A) étant égal à 50%.

12. Dispersion selon la revendication 10 ou la revendication 11, **caractérisée en ce qu'**elle présente une concentration en métal M et en le (ou les) élément(s) supplémentaire(s) d'au moins 0,5 mole/l.

13. Dispersion selon l'une quelconque des revendications 10 à 12, **caractérisée en ce que** le taux de complexant au sein de ladite dispersion exprimé en nombre de moles de complexant par rapport au nombre de moles de métal M et dudit (ou desdits) élément(s) supplémentaire(s), est compris entre 0,1 et 2.

14. Dispersion selon l'une quelconque des revendications 10 à 13, **caractérisée en ce que** le taux de complexant présent dans les colloïdes exprimé en nombre de moles de complexant par rapport au nombre de moles de métal M et dudit (ou desdits) élément(s) supplémentaire(s), est compris entre 0,1 et 2.

15. Procédé de préparation d'une dispersion selon l'une quelconque des revendications 1 à 9, **caractérisé en ce qu'**il comporte les étapes consistant à :

(a) former un mélange aqueux comprenant au moins un sel dudit métal M, et au moins un complexant, avec un taux molaire de complexant par rapport au(x) sel(s) introduit(s) compris entre 0,1 et 3;

(b) ajouter une base au mélange ainsi formé ; et

(c) chauffer le mélange ainsi obtenu, ce par quoi on obtient la dispersion.

**16.** Procédé de préparation d'une dispersion selon l'une quelconque des revendications 10 à 14, **caractérisé en ce qu'**il comporte les étapes consistant à :

(a) former un mélange aqueux comprenant au moins un sel dudit métal M, un ou plusieurs sels dudit (ou desdits) élément(s) métalliques(s) additionnel(s), et au moins un complexant, avec un taux molaire de complexant par rapport au(x) sel(s) introduit(s) compris entre 0,1 et 3 ;
(b) ajouter une base au mélange ainsi formé ; et
(c) chauffer le mélange ainsi obtenu, ce par quoi on obtient la dispersion.

**17.** Procédé de préparation d'une dispersion selon l'une quelconque des revendications 10 à 14, **caractérisé en ce qu'**il comporte les étapes consistant à:

(a) former :

(i) d'une part un mélange aqueux comprenant au moins un sel dudit métal M, et un premier complexant ; et
(ii) d'autre part un ou plusieurs autre(s) mélange(s) comprenant chacun un ou plusieurs sel(s) dudit ou desdits ou élément(s) métallique(s) supplémentaire(s) et un complexant identique ou différent de celui du mélange aqueux (i),

avec un taux molaire de complexant par rapport au(x) sel(s) introduit(s) compris entre 0,1 et 3 ;
(b) ajouter une base à chacun des mélanges ainsi formé, puis réunir ces mélanges et ajuster éventuellement le pH; et
(c) chauffer le milieu aqueux ainsi obtenu, ce par quoi on obtient la dispersion.

**18.** Procédé selon l'une quelconque des revendication 15 à 17, **caractérisé en ce que** dans le ou les mélanges réalisés dans l'étape (a), le taux molaire de complexant par rapport au(x) sel(s) introduit(s) est compris entre 0,25 et 2.

**19.** Procédé selon l'une quelconque des revendication 15 à 18, **caractérisé en ce** l'étape (c) de traitement thermique est conduite à une température d'au moins 60°C, et de préférence d'au moins 100°C.

**20.** Procédé selon l'une quelconque des revendication 15 à 19, **caractérisé en ce qu'**on soumet la dispersion obtenue à l'issue de l'étape (c) à un traitement d'ultrafiltration.

**21.** Procédé selon l'une quelconque des revendication 15 à 20, **caractérisé en ce qu'**on soumet en outre la dispersion à une étape de concentration par évaporation.

**22.** Utilisation d'une dispersion selon l'une des revendications 1 à 14 ou d'une dispersion obtenue par le procédé de l'une quelconque des revendications 15 à 21, en catalyse pour une réaction de trans-estérification ou de polycondensation, ou encore pour une réaction d'amidification, dans la préparation de films de polymères, dans une composition cosmétique, ou comme renfort pour une matrice minérale ou polymère.

## Claims

**1.** Aqueous colloidal dispersion, within which the mean diameter of the colloidal particles is at most 6 nm, said dispersion comprising:

(1) at least one compound of a metal M, said metal M being a metal in the majority within the dispersion, chosen from the group comprising aluminium, zirconium and metals of the periodic table, the atomic number of which is between 22 and 31;

(2) at least one complexing agent having a $pK_s$(M) (cologarithm of the dissociation constant of the complex formed by the complexing agent and the cation of said metal M) greater than 2.

**2.** Dispersion according to claim 1, **characterised in that** the colloidal particles have a mean diameter between 2 and 5 nm.

**3.** Dispersion according to claim 1 or claim 2, **characterised in that** the metal M is chosen from aluminium, titanium, iron and zirconium.

**4.** Dispersion according to one of the preceding claims, **characterised in that** the complexing agent is chosen from acid-alcohols or polyacid-alcohols, aminated aliphatic acids, aminated aliphatic polyacids, polyacrylic acids or the salts of these compounds.

**5.** Dispersion according to one of the preceding claims, **characterised in that** the complexing agent has a $pK_s$ of at least 3.

**6.** Dispersion according to one of the preceding claims, **characterised in that** it has a pH between 3 and 10.

**7.** Dispersion according to one of the claims I to 6, **characterised in that** it has a concentration of metal M of at least 0.5 mole/l.

**8.** Dispersion according to one of the preceding claims, **characterised in that** the ratio of complexing agent within the suspension, expressed as the number of moles of complexing agent relative to the number of moles of metal M, is between 0.1 and 2.

**9.** Dispersion according to one of the preceding claims, **characterised in that** the ratio of complexing agent present in the colloids, expressed as the number of moles of complexing agent relative to the number of moles of metal M, is between 0.1 and 2.

**10.** Dispersion according to one of the claims 1 to 9, **characterised in that** it comprises one or more compound(s) of one or more additional metallic element(s) other than said metal M, chosen from the lanthanides, aluminium and the elements of the groups IVa, Va, VIa, VIIa, VIII, Ib and IIb of the periodic table, the molar proportion (metal M) /(metal M + other element(s)) being strictly greater than 50%.

**11.** Dispersion according to one of the claims 1 to 9, **characterised in that** it comprises a compound of an additional metallic element A, chosen from a luminium and the elements of the groups IVa, Va, VIa, VIIa, VIII, Ib and IIb of the periodic table, the molar proportion M/(M + A) being equal to 50%.

**12.** Dispersion according to claim 10 or claim 11, **characterised in that** it has a concentration of metal M and of the supplementary element(s) of at least 0.5 mole/l.

**13.** Dispersion according to one of the claims 10 to 12, **characterised in that** the ratio of complexing agent within said dispersion, expressed as the number of moles of complexing agent relative to the number of moles of metal M and said supplementary element(s), is between 0.1 and 2.

**14.** Dispersion according to any of the claims 10 to 13, **characterised in that** the ratio of complexing agent present in the colloids, expressed as the number of moles of complexing agent relative to the number of moles of metal M and of said supplementary element(s), is between 0.1 and 2.

**15.** Method of preparing a dispersion according to any of the claims 1 to 9, **characterised in that** it includes the steps comprising:

(a) forming an aqueous mixture comprising at least one salt of said metal M, and at least one complexing agent, with a molar ratio of complexing agent relative to the introduced salt(s) of between 0.1 and 3;

(b) adding a base to the mixture thus formed; and

(c) heating the mixture thus obtained, by means of which the dispersion is obtained.

**16.** Method of preparing a dispersion according to any of the claims 10 to 14, **characterised in that** it includes the steps comprising:

(a) forming an aqueous mixture comprising at least one salt of said metal M, one or more of said salts of said additional metallic element(s), and at least one complexing agent, with a molar ratio of complexing agent

relative to the introduced salt(s) of between 0.1 and 3;

(b) adding a base to the mixture thus formed; and

(c) heating the mixture thus obtained, by means of which the dispersion is obtained.

**17.** Method of preparing a dispersion according to any of the claims 10 to 14, **characterised in that** it includes the steps comprising:

(a) forming:

(i) on the one hand, an aqueous mixture comprising at least one salt of said metal M and a first complexing agent; and
(ii) on the other hand, one or more other mixture(s) each comprising one or more salt(s) of said supplementary metallic element(s) and a complexing agent identical to or different from that of the aqueous mixture (i),

with a molar ratio of complexing agent relative to the introduced salt(s) of between 0.1 and 3;

(b) adding a base to each of the mixtures thus formed, then reuniting these mixtures and possibly adjusting the pH; and

(c) heating the aqueous medium thus obtained, by means of which the dispersion is obtained.

**18.** Method according to any of the claims 15 to 17, **characterised in that**, in the mixture(s) produced in step (a), the molar ratio of complexing agent relative to the introduced salt(s) is between 0.25 and 2.

**19.** Method according to any of the claims 15 to 18, **characterised in that** the thermal treatment step (c) is conducted at a temperature of at least 60°C, and preferably of at least 100°C.

**20.** Method according to any of the claims 15 to 19, **characterised in that** the dispersion obtained at the end of step (c) is subjected to an ultrafiltration treatment.

**21.** Method according to any of the claims 15 to 20, **characterised in that** the dispersion is furthermore subjected to a step of concentration by evaporation.

**22.** Use of a dispersion according to any of the claims 1 to 14 or of a dispersion obtained by the method of any of the claims 15 to 21, in catalysis for a transestcrification or polycondensation reaction, or moreover for an amidation reaction, in the preparation of polymer films, in a cosmetic composition or as a reinforcement for a mineral or polymer matrix.

**Patentansprüche**

**1.** Wässrige kolloide Dispersion, in welcher der mittlere Durchmesser der kolloiden Teilchen höchstens 6 nm beträgt, und welche:

1) mindestens eine Verbindung eines Metalls M, das in der Dispersion ein überwiegendes Metall ist und aus der aus Aluminium, Zirconium und Metallen des Periodensystems, deren Ordnungszahl 22 bis 31 beträgt, bestehenden Gruppe ausgewählt ist, und

2) mindestens ein Komplexierungsmittel, das einen $pK_s(M)$ (negativer dekadischer Logarithmus der Dissoziationskonstante des von dem Komplexierungsmittel und dem Kation des Metalls M gebildeten Komplexes) von größer als 2 besitzt,

umfasst.

**2.** Dispersion nach Anspruch 1, **dadurch gekennzeichnet, dass** die kolloiden Teilchen einen mittleren Durchmesser

von 2 bis 5 nm besitzen.

3. Dispersion nach Anspruch 1 oder 2, **dadurch gekennzeichnet, dass** das Metall M aus Aluminium, Titan, Eisen und Zirconium ausgewählt ist.

4. Dispersion nach einem der vorhergehenden Ansprüche, **dadurch gekennzeichnet, dass** das Komplexierungsmittel aus Hydroxycarbonsäuren bzw. Hydroxypolysäuren, aliphatischen Aminosäuren, aliphatischen Polyaminosäuren, Polyacrylsäuren oder Salzen dieser Verbindungen ausgewählt ist.

5. Dispersion nach einem der vorhergehenden Ansprüche, **dadurch gekennzeichnet, dass** der $pK_s$ des Komplexierungsmittels mindestens 3 beträgt.

6. Dispersion nach einem der vorhergehenden Ansprüche, **dadurch gekennzeichnet, dass** ihr pH-Wert 3 bis 10 beträgt.

7. Dispersion nach einem der Ansprüche 1 bis 6, **dadurch gekennzeichnet, dass** ihre Konzentration des Metalls M mindestens 0,5 mol/l beträgt.

8. Dispersion nach einem der vorhergehenden Ansprüche, **dadurch gekennzeichnet, dass** der Anteil des Komplexierungsmittels an der Suspension, angegeben als Anzahl Mole des Komplexierungsmittels, bezogen auf die Anzahl Mole des Metalls M, 0,1 bis 2 beträgt.

9. Dispersion nach einem der vorhergehenden Ansprüche, **dadurch gekennzeichnet, dass** der Anteil des Komplexierungsmittels an den Kolloiden, angegeben als Anzahl Mole des Komplexierungsmittels, bezogen auf die Anzahl Mole des Metalls M, 0,1 bis 2 beträgt.

10. Dispersion nach einem der Ansprüche 1 bis 9, **dadurch gekennzeichnet, dass** sie eine oder mehrere Verbindungen eines oder mehrerer zusätzlicher metallischer Elemente enthält, das/die nicht das Metall M ist/sind und aus den Lanthanoiden, Aluminium und den Elementen der Gruppen IVa, Va, VIa, VIIa, VIII, Ib und IIb des Periodensystems ausgewählt ist/sind, wobei das Molverhältnis von (Metall M)/(Metall M + andere/s Element/e) streng größer als 50 % ist.

11. Dispersion nach einem der Ansprüche 1 bis 9, **dadurch gekennzeichnet, dass** sie eine Verbindung eines zusätzlichen metallischen Elements A enthält, das aus Aluminium und den Elementen der Gruppen IVa, Va, VIa, VIIa, VIII, Ib und IIb des Periodensystems ausgewählt ist, wobei das Molverhältnis von M/(M + A) gleich 50 % ist.

12. Dispersion nach Anspruch 10 oder 11, **dadurch gekennzeichnet, dass** ihre Konzentration des Metalls M und des/der zusätzlichen Elemente/s mindestens 0,5 mol/l beträgt.

13. Dispersion nach einem der Ansprüche 10 bis 12, **dadurch gekennzeichnet, dass** der Anteil des Komplexierungsmittels an der Dispersion, angegeben als Anzahl Mole des Komplexierungsmittels, bezogen auf die Anzahl Mole des Metalls M und des/der zusätzlichen Elemente/s, 0,1 bis 2 beträgt.

14. Dispersion nach einem der Ansprüche 10 bis 13, **dadurch gekennzeichnet, dass** der Anteil des Komplexierungsmittels an den Kolloiden, angegeben als Anzahl Mole des Komplexierungsmittels, bezogen auf die Anzahl Mole des Metalls M und des/der zusätzlichen Elemente/s, 0,1 bis 2 beträgt.

15. Verfahren zur Herstellung einer Dispersion nach einem der Ansprüche 1 bis 9, **dadurch gekennzeichnet, dass** es die Stufen umfasst, die darin bestehen:

   a) ein wässriges Gemisch zu bilden, das mindestens ein Salz des Metalls M und mindestens ein Komplexierungsmittel enthält, wobei das Molverhältnis von Komplexierungsmittel zu dem/den zugesetzten Salz/en 0,1 bis 3 beträgt,

   b) dem so gebildeten Gemisch eine Base zuzugeben und

   c) das so resultierende Gemisch zu erwärmen, wodurch die Dispersion erhalten wird.

**16.** Verfahren zur Herstellung einer Dispersion nach einem der Ansprüche 10 bis 14, **dadurch gekennzeichnet, dass** es die Stufen umfasst, die darin bestehen:

a) ein wässriges Gemisch zu bilden, das mindestens ein Salz des Metalls M, ein oder mehrere Salze des/der zusätzlichen metallischen Elemente/s und mindestens ein Komplexierungsmittel enthält, wobei das Molverhältnis von Komplexierungsmittel zu dem/den zugesetzten Salz/en 0,1 bis 3 beträgt,

b) dem so gebildeten Gemisch eine Base zuzugeben und

c) das so resultierende Gemisch zu erwärmen, wodurch die Dispersion erhalten wird.

**17.** Verfahren zur Herstellung einer Dispersion nach einem der Ansprüche 10 bis 14, **dadurch gekennzeichnet, dass** es die Stufen umfasst, die darin bestehen:

a)

I) einerseits ein wässriges Gemisch, das mindestens ein Salz des Metalls M und ein erstes Komplexierungsmittel enthält, und
II) andererseits ein oder mehrere weitere Gemische, die jeweils ein oder mehrere Salze des/der zusätzlichen metallischen Elemente/s und ein Komplexierungsmittel enthalten, das gegebenenfalls ein anderes als das des wässrigen Gemischs I) ist,
wobei das Molverhältnis von Komplexierungsmittel zu dem/den zugesetzten Salz/en 0,1 bis 3 beträgt,

zu bilden,

b) jedem der so gebildeten Gemische eine Base zuzugeben, anschließend diese Gemische zu vereinigen und gegebenenfalls den pH-Wert einzustellen und

c) das so resultierende wässrige Medium zu erwärmen, wodurch die Dispersion erhalten wird.

**18.** Verfahren nach einem der Ansprüche 15 bis 17, **dadurch gekennzeichnet, dass** das Molverhältnis von Komplexierungsmittel zu dem/den zugesetzten Salz/en des/der in Stufe a) hergestellten Gemische/s 0,25 bis 2 beträgt.

**19.** Verfahren nach einem der Ansprüche 15 bis 18, **dadurch gekennzeichnet, dass** die Stufe c) zur Wärmebehandlung bei einer Temperatur von mindestens 60 °C und vorzugsweise mindestens 100 °C durchgeführt wird.

**20.** Verfahren nach einem der Ansprüche 15 bis 19, **dadurch gekennzeichnet, dass** die nach Stufe c) erhaltene Dispersion einer Ultrafiltration unterworfen wird.

**21.** Verfahren nach einem der Ansprüche 15 bis 20, **dadurch gekennzeichnet, dass** die Dispersion außerdem einer Stufe unterworfen wird, in welcher sie durch Eindampfen aufkonzentriert wird.

**22.** Verwendung einer Dispersion nach einem der Ansprüche 1 bis 14 oder einer Dispersion, die durch das Verfahren nach einem der Ansprüche 15 bis 21 erhalten worden ist, zur Katalyse einer Umesterung, Polykondensation oder Amidierung, zur Herstellung von Polymerfilmen, in einer kosmetischen Zusammensetzung oder als Verstärkung einer anorganischen oder polymeren Matrix.